# FASCICULE DE BREVET EUROPEEN

(11) **EP 1 044 966 B1**
(45) Date de publication et mention de la délivrance du brevet: **22.09.2004**
(21) Numéro de dépôt: 00401002.1
(22) Date de dépôt: 11.04.2000
(51) Int. Cl.: C07C 323/52, C07C 323/12, C07D 257/04, C07D 263/10, A61K 31/19, A61K 31/10, A61K 31/41, A61K 31/42

(54) **Composés (poly)thia-alcynoiques et leurs dérivés, compositions les comprenant et leur utilisation**
(Poly)thia-alkin-Verbindungen und ihre Derivate, diese enthaltende Zusammensetzungen und ihre Anwendung
(Poly)thia-alkyne compounds and their derivatives, compositions containing them and their use

(30) Priorité: 15.04.1999 FR 9904745
(43) Date de publication de la demande: 18.10.2000
(73) Titulaire: L'OREAL, 75008 Paris (FR)
(72) Inventeur: Maignan, Jean, 93290 Tremblay en France (FR); Michel, Serge, 06330 Roquefort Les Pins (FR); Genard, Sylvie, 75012 Paris (FR)
(74) Mandataire: Renard, Emmanuelle

(56) Documents cités:
- EP-A- 0 342 115
- DATABASE CHEMABS [Online] CHEMICAL ABSTRACTS SERVICE, COLUMBUS, OHIO, US STN, CAPLUS accession no. 1975:155358, XP002129417 -& JP 49 102616 A (MEIJI CONFECTIONARY CO LTD)

## Description

L'invention concerne, à titre de produits industriels nouveaux et utiles, des composés (poly)thia-alcynoïques. Elle concerne également l'utilisation de ces nouveaux composés dans des compositions pharmaceutiques destinées à un usage en médecine humaine ou vétérinaire, ou bien encore dans des compositions cosmétiques.

Ces composés de formule générale (I), conformes à l'invention ont une activité vis-à-vis de la transactivation des récepteurs de type PPARs et plus particulièrement des récepteurs du sous-type PPAR-α et trouvent des applications en particulier dans le traitement des affections inflammatoires telles que l'arthrite rhumatoïde, le lupus et le psoriasis notamment.

On peut également utiliser les composés selon l'invention dans des compositions cosmétiques pour l'hygiène corporelle et capillaire en particulier pour réguler le métabolisme des lipides cutanés, restaurer la fonction barrière cutanée ou promouvoir la différentiation et inhiber la prolifération épidermique.

Il est connu qu'un certain nombre de substances jouent un rôle important dans le processus inflammatoire de la peau telles que l'acné, les dermatoses, comme par exemple le psoriasis, l'eczéma, etc. Ces substances, parmi lesquelles les prostaglandines, les acides hydroxyeicosatétraénoïques, les thromboxanes et les leucotriènes, ont toutes une origine commune qui est l'acide arachidonique (voir en particulier « VOORHEES-Leukotrienes and other Lipoxygenase Products in the Pathogenesis and Therapy of Psoriasis and Other Dermathoses » Arch. Dermatol., Vol. 119, Juillet 1983, 541-547).

La formation de ces substances résulte essentiellement de la transformation après libération de l'acide arachidonique lié par une liaison ester aux lipides présents dans l'épiderme (par exemple les phospholipides).

On a déjà préconisé antérieurement, pour le traitement des maladies de la peau, soit des inhibiteurs de la cyclooxygénase empêchant la formation des prostaglandines tels que l'indométhacine, la vitamine E, etc... ; ou alors des substances susceptibles d'inhiber les lipoxygénases tels que l'acide eicosatétraynoïque.

On a également proposé pour le traitement du psoriasis l'acide eicosatétraynoïque-5, 8, 11, 14 ainsi que l'acide eicosatriynoïque-5, 8, 11 et leurs esters d'alkyle inférieurs, notamment dans le brevet US-A-4 190 669 ou bien le remplacement du groupement méthylène en position-3 dans la structure de l'acide eicosatriynoïque-5, 8, 11 ou de l'acide eicosatétraynoïque-5, 8, 11, 14, par un hétéroatome tel que le soufre ou par un groupement sulfoxyde ou sulfone, notamment dans le brevet EP 342 115.

La demanderesse a découvert que, de façon surprenante, en raccourcissant la longueur de la chaîne des acides gras insaturés de type thiaeicosa(poly)ynoïque, on obtenait des produits activateurs des récepteurs de type PPARs et plus particulièrement des activateurs sélectifs pour un sous-type de récepteurs PPAR-α.

Ces acides présentent en outre l'avantage d'être d'un prix de revient beaucoup plus intéressant que leurs homologues à chaîne plus longue.

La demanderesse a découvert également que, de façon surprenante, en remplaçant le groupement méthylène en position-8 dans la chaîne de l'acide gras thia-3 insaturé par un hétéroatome tel que le soufre ou par un groupement sulfoxyde ou sulfone, on obtenait également des activateurs des récepteurs de type PPARs et plus particulièrement des activateurs sélectifs pour un sous-type de récepteurs PPAR-α.

L'invention a donc pour objet ces nouveaux acides.

Les composés selon l'invention peuvent être représentés par la formule générale (I) suivante :

**R**_{**1**}**-Y-CH**_{**2**}**-C≡C-CH**_{**2**}**-S-CH**_{**2**}**-R**_{**2**} **(I)**

dans laquelle :
- Y représente :
   (a) un radical -S(O)t,
      t est un nombre entier égal à 0, 1 ou 2,
   (b) un radical -CH₂-,
   (c) un radical -C ≡ C-,
   (d) un radical -C = C-,
- R₁ représente un radical alkyle linéaire ou ramifié ayant de 1 à 18 atomes de carbone éventuellement substitué par un ou plusieurs atomes d'halogène, un radical alkényle linéaire ou ramifié ayant de 1 à 18 atomes de carbone, ou un radical alkynyle linéaire ou ramifié ayant de 1 à 18 atomes de carbone, ce radical pouvant en outre comprendre un ou plusieurs atomes d'oxygène et/ou atomes d'azote et/ou atomes de soufre,
étant entendu que :
- lorsque Y représente (b), alors R₁ comprend un nombre d'atomes compris entre 1 et 12 inclusivement, et de préférence compris entre 4 et 12 inclusivement, et de manière encore plus préférentielle entre 6 et 12 inclusivement,
- lorsque Y représente (c), alors R₁ comprend un nombre d'atomes compris entre 1 et 10 inclusivement, et de préférence compris entre 4 et 10 inclusivement, et de manière encore plus préférentielle entre 6 et 10 inclusivement,
- lorsque Y est différent de (b) et que R₁ est un radical insaturé ou comporte un hétéroatome, alors l'insaturation et/ou l'hétéroatome de R₁ ne peuvent pas être en position α par rapport à Y,
- R₂ représente :
   (e) un radical -CO-R₄,
- R₄ représente :
   un radical -OR₅
- R₅ représente :
   un atome d'hydrogène,

L'invention vise également les sels des composés de formule (I) lorsque R₂ représente une fonction acide carboxylique et les isomères géométriques et optiques desdits composés de formule (I).

Lorsque les composés selon l'invention se présentent sous forme de sels par addition d'une base, il s'agit de sels d'un métal alcalin ou alcalino-terreux, ou encore de sels de zinc, de magnésium ou de strontium, d'une amine organique ou les sels d'ammonium quatemaires, lorsqu'ils comportent au moins une fonction acide libre.

Lorsque les composés de l'invention se présentent sous forme de sels, par addition d'un acide, il s'agit de sels pharmaceutiquement ou cosmétiquement acceptables obtenus par addition d'un acide minéral ou organique, en particulier l'acide chlorhydrique, bromhydrique, sulfurique, acétique, citrique, fumarique, hémisuccinique, maléique et mandélique.

Selon la présente invention, on entend par radical alkyle inférieur un radical linéaire ou ramifié ayant de 1 à 6 atomes de carbone et, de préférence, les radicaux méthyle, éthyle, isopropyle, n-butyle, tertiobutyle, pentyle ou hexyle.

Par radical alkyle on entend un radical linéaire ou ramifié ayant de 1 à 18 atomes de carbone éventuellement substitué par un ou plusieurs atomes d'halogène.
Parmi les atomes d'halogène, on préfère un atome de fluor, de chlore ou de brome.
Les radicaux alkyles sont choisis de préférence parmi les radicaux méthyle, éthyle, propyle, isopropyle, butyle, tertiobutyle, pentyle, hexyle, ou 2-éthyl-hexyle, octyle, nonyle, décyle, dodécyle, dodécanyle, tétradécanyle ou tridécafluoro-3,3,4,4,5,5,6,6,7,7,8,8,8-octyle.

Par radical alkényle on entend un radical linéaire ou ramifié ayant de 1 à 18 atomes de carbone comportant une ou plusieurs doubles liaisons et de préférence les radicaux allyle, butènyte, hexènyle, octènyle, décènyle, dodécènyle, ou tétradécènyle.

Par radical alkynyle on entend un radical linéaire ou ramifié ayant de 1 à 18 atomes de carbone comportant une à plusieurs triple liaisons et de préférence les radicaux propynyle, butyne-2-yle, pentyn-2-yle, hexyn-2-yle, octyn-2-yne, décyn-2-yle, ou dodécyn-2yle-2.

Parmi les composés de formule (I) ci-dessus rentrant dans le cadre de la présente invention, on peut notamment citer les suivants :
- l'acide tridécafluoro-11,11,12,12,13,13,14,14,15,15,16,16,16 dithia-3,8 hexadécyne-5oïque,
- l'acide dithia-3,8 docosyne-5oïque,
- le dithia-3,8 hexadécyne-5oate de méthyle,
- l'acide dithia-3,8 hexadécyne-5oïque,
- l'acide thia-3 hexadécyne-5oïque,
- l'acide dithia-3,8 heptadécyne-5oïque,
- l'acide thia-3 heptadécadiyne-5,8oïque,
- l'acide thia-3 octadécadiyne-5,8oïque,
- l'acide thia-3 pentadécadiyne-5,8oïque,
- l'acide thia-3octadecatriyne-5,8,11oïque,
- l'acide thia-3octadecayne-5oïque,
- l'acide thia-3heptadecatriyne-5,8,11oïque,
- l'acide thia-3heptadecayne-5oïque,
- l'acide thia-3hexadecatriyne-5,8,11oïque,
- l'acide thia-3hexadecadiyne-5,8oïque,
- l'acide thia-3pentadecatriyne-5,8,11oïque,
- l'acide thia-3pentadecayne-5oïque,
- l'acide thia-3tetradecayne-5oïque,
- l'acide thia-3 heptadecatriyne-5,8,11oïque

Selon la présente invention, les composés de formule (I) plus particulièrement préférés sont ceux pour lesquels au moins l'une des, et de préférence toutes les, conditions suivantes sont remplies:
- R2 est un radical -CO-R4,
- R4 est un radical hydroxyle,
- Y est choisi parmi
   - le radical (c) et R1 est un radical alkyle ayant de 4 à 10 atomes de carbone,
      ou le radical (a) dans lequel t égal 0 et R1 est un radical alkyle ayant de 4 à 12 atomes de carbone,
      ou le radical (b) et R1 est un radical fluoré ayant de 4 à 12 atomes de carbone,

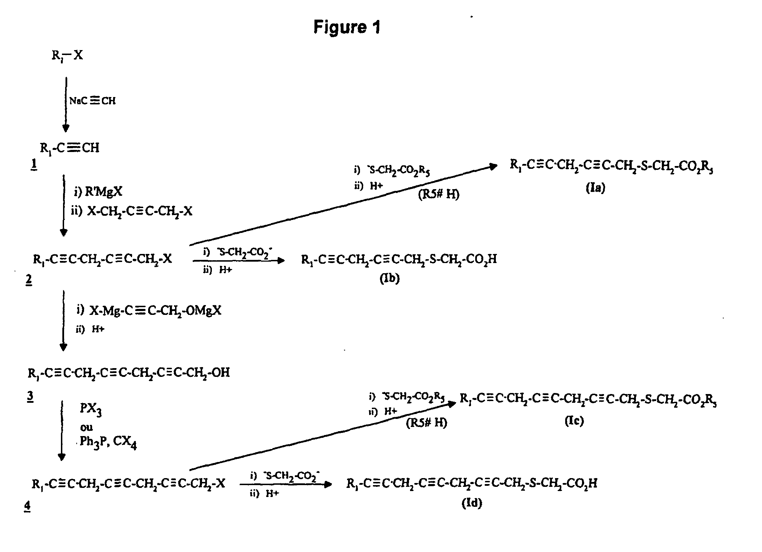

Les procédés de préparation des composés de formule (I), suivent les schémas réactionnels donnés aux **figures 1, 2, 3, 4 5, 6 et 7**.

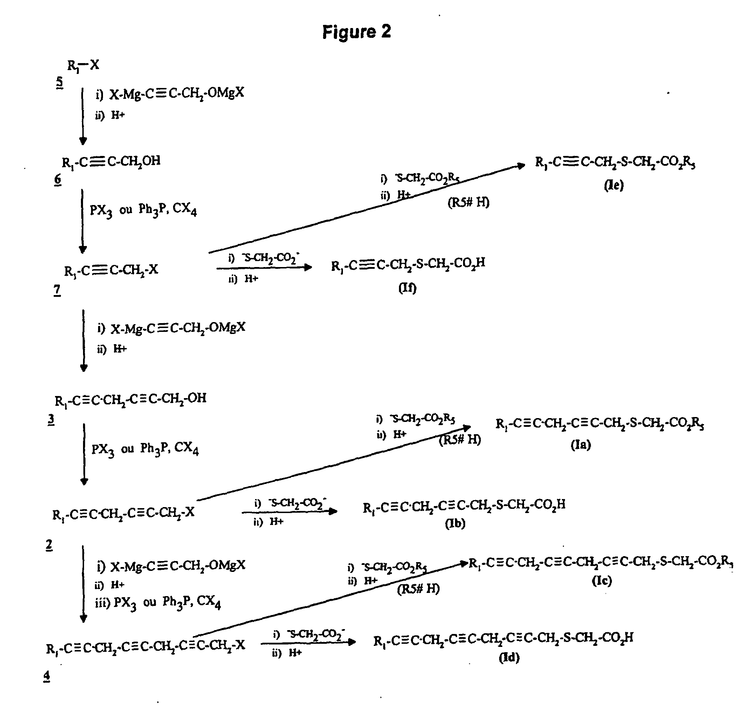

Ainsi lorsque Y correspond à un méthylène ou à une triple liaison, les composés de formule (**I**) conformes à l'invention peuvent être préparés par mise en oeuvre de l'un des deux procédés représentés aux **figures 1 et 2**.

Le premier procédé (**figure 1**) consiste à préparer l'anion d'un alcyne de formule (**1**) avec une base forte tel qu'un halogénomagnésien d'alkyle puis à le faire réagir avec un excès de dihalogéno-1,4-butyne pour former le dérivé 1-halogéno-2,5-diyne (**2**). Certains alcynes sont commerciaux comme par exemple l'heptyne-1 ou le décyne-1. Les autres alcynes de formule R₁-C≡C-H sont préparés en faisant réagir l'acétylure de sodium sur l'halogénure R₁-X correspondant.
Les dérivés triynes-2,5,8 (**4**) sont obtenus par réaction du dérivé (**2**) avec le dianion de l'alcool propargylique. L'alcool triyne (**3**) ainsi obtenu est transformé en halogénure correspondant pour conduire au dérivé 1-halogéno-2,5,8-triyne de structure (**4**).
Les halogénures alcyniques (**2**) ou (**4**) conduisent par traitement avec le dianion de l'acide thioglycolique ou avec le thiolate d'un mercaptan aux composés de l'invention de formule (**I**) pour lesquels Y correspond à une triple liaison , R₁ étant soit un radical alkyle saturé soit un radical alkyle comportant une insaturation en particulier une liaison acétylénique située en β de Y, soit encore un radical alkyle perfluoré.

Le dianion de l'acide thioglycolique est formé en traitant ce dernier avec 2 équivalents de base. Le thiolate d'un mercaptan est préparé avec un équivalent de base. Cette base est minérale ou organique, les bases préférées étant la soude, la potasse ou le méthylate de sodium.

Après réaction du dianion de l'acide thioglycolique sur l'halogénure d'alcyne, l'acide thia-3-alcynoïque de formule (**I**) est purifié par cristallisation dans un solvant approprié lorsque c'est un solide à température ordinaire, ou par chromatographie sur gel de silice pour un composé liquide à cette température. Après réaction de l'anion d'un alkylmercaptan avec l'halogénure d'alcyne, l'ester de l'acide thia-3-alcynoïque obtenu est généralement purifié par chromatographie sur colonne de silice.

Le deuxième procédé (**figure 2**) consiste à préparer directement des intermédiaires alcyniques dont la triple liaison est en position 2 par rapport à la fonction présente en bout de chaîne.

Le "chaînon propyne" est greffé par l'intermédiaire de l'alcool propargylique sur un halogénure d'alkyle de formule **5** lorsque l'alcool **6** n'est pas commercial. L'alcool alcynique **6** est transformé en halogénure **7** correspondant lorsque **7** n'est pas commercial. L'élongation de la chaîne est réalisée en greffant le dianion de l'alcool propargylique. L'alcool obtenu est ensuite transformé en halogénure correspondant **2** qui peut également être obtenu selon la figure 1. Cet halogénure **2** traité par le dianion de l'alcool propargylique conduit à l'alcool **3** qui est à son tour transformé en halogénure **4**.
Par exemple, la préparation de l'halogéno-1-tétradécadiyne-2,5 est décrite dans le brevet français 2 584 400.
Le dianion de l'alcool propargylique est préparé en traitant cet alcool par 2 équivalents basiques. Les bases utilisées sont des bases fortes telles que les organolithiens comme par exemple le n-butyllithium ou des organomagnésiens tels que l'halogénomagnésien d'éthyle ou de propyle dans un solvant anhydre, de préférence un éther comme un tétrahydrofuranne ou le diéthyl éther. Après réaction de ce dianion et acidification du milieu réactionnel, l'alcool alcynique est purifié par distillation ou recristallisation. Cet alcool est traité dans un solvant chloré tel que le dichlorométhane ou le dichloro-1,2-éthane, ou un éther, par un trihalogénure de phosphore ou un mélange triphénylphosphine, tétrahalogénure de carbone. L'halogénure d'alcyne ainsi obtenu est purifié, selon son mode de préparation, par distillation (lorsque sa stabilité le permet), ou par chromatographie.

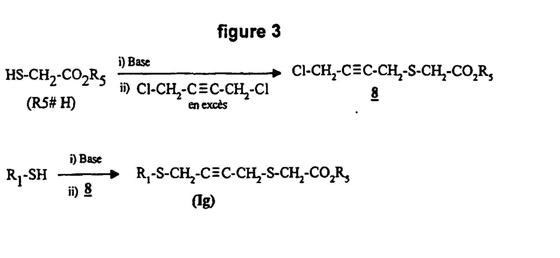

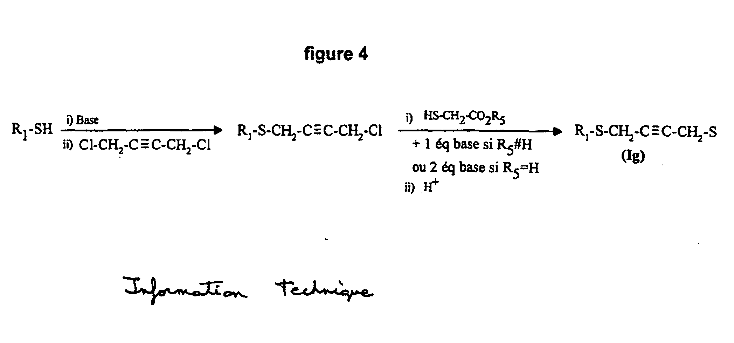

Ainsi, lorsque Y correspond à atome de soufre, les composés de formule (**I**) conformes à l'invention peuvent être préparés par mise en oeuvre de l'un des deux procédés représentés **aux figures 3 et 4**.

Le premier procédé (**figure 3**) consiste à préparer les composés de invention à partir de l'ester **8** obtenu par réaction de l'anion du thioglycolate d'alkyle tel que le thioglycolate de méthyle, que l'on fait réagir avec le dichloro-1,4-butyne utilisé en excès de façon à favoriser la réaction de monosubstitution. L'halogénoester **8** ainsi obtenu est alors mis en réaction avec les anions des mercaptans de structure R₁-SH. Ces réactions sont réalisées dans des solvants dipolaires usuels tels que les alcools comme le méthanol ou les éthers comme le tétrahydrofuranne.

Il est entendu que le thiolate R₁S⁻ peut réagir sur un excès de 1,4-dichloro-2-butyne pour former l'alcyne R₁-S-CH₂-C≡C-CH₂-Cl pouvant à son tour réagir avec le dianion de l'acide thioglycolique ou le thiolate d'un mercaptan pour former les dérivés de structure (**l**)(**figure 4**).

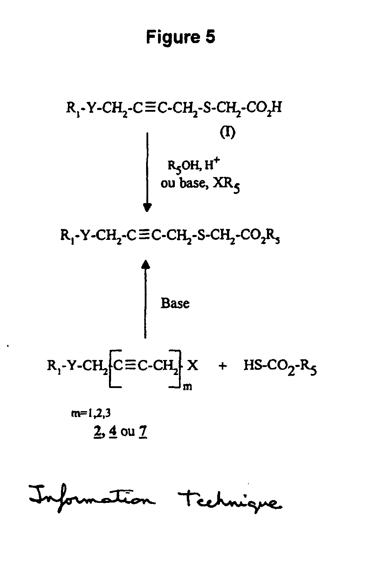

Les acides carboxyliques de structure (**I**) peuvent être transformés en esters correspondants suivant les méthodes usuelles de transformation d'un acide carboxylique en ester, c'est à dire par réaction d'un alcool en milieu acide ou par réaction de déplacement de l'halogène d'un halogénure d'alkyle par la fonction carboxylate de sodium ou de potassium de l'acide (**I**) ou encore en faisant réagir une forme activée des acides de formule (**I**) sur un alcool R₅-OH. Par forme activée, on entend l'intermédiaire formé, par addition à une solution d'acide, de carbonyldiimidazole (CDI), de dicyclohexylcarbodiimide (DCC) ou de tout autre réactif destiné à former une forme activée d'acide, choisi parmi ceux connus dans la littérature(**figure 5**).

Une autre voie de préparation est de faire réagir le thiolate d'un thioglycolate d'alkyle traité par 1 équivalent de base sur un halogénure de formule **2**, **4** ou **7**.

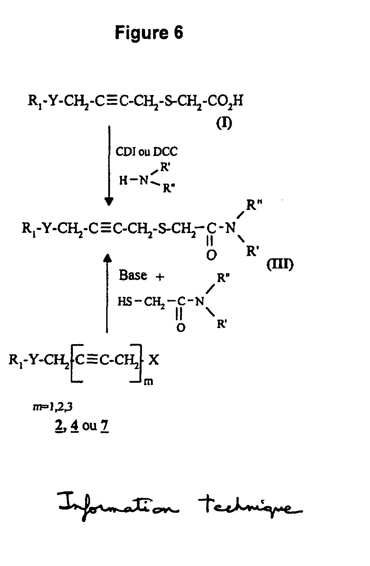

Les amides rentrant dans la définition de la formule générale (**I**) dans laquelle R₂ désigne le groupe COR₄ et R₄ le radical amino -NR'(R") conformes à l'invention, sont obtenus en faisant réagir une forme activée des acides de formule (**I**) sur une amine dans un solvant organique. Cette forme activée de l'acide peut être, soit un chlorure d'acide, soit un anhydride ou encore l'intermédiaire formé par addition à une solution d'acide, de carbonyldiimidazole (CDI), de dicyclohexylcarbodiimide (DCC) ou de tout autre réactif destiné à former une forme activée d'acide, choisi parmi ceux connus dans la littérature. Cette dernière réaction est conduite de préférence en milieu solvant tel que le diméthylformamide ou encore un solvant chloré comme le dichlorométhane ou le dichloro-1,2-éthane. Cette réaction se déroule selon le schéma réactionnel donné à la **figure 6**.

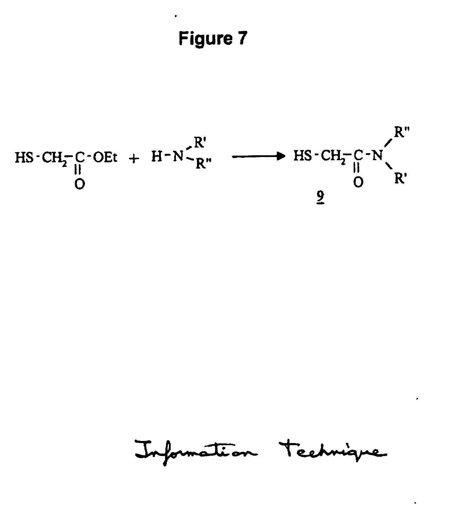

Lorsque les thioglycolamides sont facilement accessibles, les amides peuvent être obtenus directement sans passer par l'intermédiaire ce l'acide de formule (**I**) en traitant les halogénures **2**, **4** ou **7** par le thiolate formé au préalable à partir du thioglycolamide **9**. Ce dernier est préparé par action d'une amine H-NR'(R") sur le thioglycolate d'éthyle HS-CH₂-CO₂Et (**figure 7**).
Cette méthode est en effet plus simple. On prépare les halogénures **2**, **4** ou **7** d'une part, et le sel de potassium ou de sodium du thioglycolamide **9** d'autre part, dans le méthanol ou l'éthanol. Les halogénures **2**, **4** ou **7** ne sont pas purifiés et leur mélange réactionnel est directement ajouté à une solution du thioglycolamide salifié par 1 équivalent de base.

Les composés de l'invention présentent des propriétés d'activation des récepteurs de type PPARs. Plus particulièrement les composés de l'invention présentent des propriétés d'activation sélective des récepteurs du sous-type PPAR-α.

Par activateur des récepteurs de type PPAR-α, on entend selon l'invention tout composé qui présente dans un test de transactivation, tel que décrit dans Kliewer et al., Nature 358, 771-774, 1992, une AC50 relative au PPAR-α inférieure ou égale à 10 µM. De préférence, l'activateur des récepteurs de type PPAR-α présente une AC50 relative au PPAR-α inférieure ou égale à 3,5 µM et avantageusement inférieure ou égale à 3 µM.

De préférence, l'activateur des récepteurs de type PPAR-α est sélectif, c'est à dire qu'il présente un rapport R1 d'AC50 relative au PPAR-α sur l'AC50 relative aux autres sous-types de PPAR (PPAR-δ ou PPAR-γ) inférieur ou égal à 10⁻¹. De préférence, R1 est inférieur ou égale à 0,05, et plus avantageusement inférieur ou égale à 0,02.

Une AC50 est la concentration en composé "activateur" nécessaire pour présenter 50% de l'activité d'une molécule de référence. Cette activité est déterminée à l'aide d'une enzyme (luciférase) rapporteuse de l'activation due au composé via un des récepteurs PPARs, et plus particulièrement de type PPAR-α.

L'activité des récepteurs de type PPARs et plus particulièrement des sous-type PPAR-α a fait l'objet de nombreuses études. L'ensemble des références suggèrent un rôle des récepteurs de type PPARs dans la régulation du métabolisme et l'homéostasie des lipides.
On peut citer à titre indicatif la publication intitulée "Differential Expression of Peroxisome Proliferator-Activated Receptor Subtypes During the Differentiation of Human Keratinocytes", Michel Rivier et al., J. Invest. Dermatol 111, 1998, p 1116-1121, dans laquelle sont répertoriées un grand nombre de références bibliographiques concernant les récepteurs de type PPARs.
Les PPARα sont impliqués dans le contrôle de l'inflammation.
L'utilisation des activateurs des récepteurs de type PPAR-α pour restaurer la fonction barrière, promouvoir la différentiation et inhiber la prolifération épidermique a été décrite dans la demande de brevet internationale WO 98/32444.
De plus, l'utilisation des activateurs des récepteurs de type PPAR-α et/ou PPAR-γ pour traiter les désordres cutanés liés à une anomalie de la différenciation des cellules épidermiques a été décrite dans la publication de Michel Rivier et al., J. Invest. Dermatol 111, 1998, p 1116-1121.
Les désordres cutanés liés à une anomalie de la différenciation des cellules épidermiques sont notamment le psoriasis, l'eczéma, le lichen plan, les lésions de la peau associées à un lupus, les dermatites, telles que les dermatites atopique, sébhorréïque ou solaire, les kératoses, telles que la kératose sébhorréïque, sénile, actinique, photo-induite ou folliculaire, l'acné vulgaire, les kéloïdes, les nevi, les verrues, les ichtyoses et les cancers cutanés.

Les composés de formule (I) selon l'invention, trouvent une application dans le domaine cosmétique, en particulier dans l'hygiène corporelle et capillaire et plus particulièrement pour réguler le métabolisme des lipides cutanés, pour le traitement des peaux à tendance acnéique, pour lutter contre l'aspect gras de la peau ou des cheveux, ou dans le traitement des peaux physiologiquement sèches.

L'utilisation d'au moins un composé de formule (I) permet également de restaurer la fonction barrière cutanée et/ou de promouvoir la différentiation et d'inhiber la prolifération épidermique. Par rapport aux produits connus antérieurement, ces composés de formule (I) ont l'avantage de présenter en plus d'autres propriétés intéressantes, notamment des propriétés anti-inflammatoires ou apaisantes, ce qui en fait des composés moins irritants et donc mieux tolérés.

La présente invention vise donc une composition cosmétique contenant, dans un support cosmétiquement acceptable, au moins un composé de formule (I) l'un de ses isomères optiques ou géométriques ou l'un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques est comprise entre 0,0001 et 3 % en poids, de préférence entre 0,001 et 1 % en poids, par rapport au poids total de la composition.

La présente invention a également pour objet à titre de médicament les composés de formule (I) tels que décrits ci-dessus.

Les composés selon l'invention conviennent particulièrement bien dans les domaines de traitement suivants :
1) les affections dermatologiques liées à une anomalie de la différenciation des cellules épidermiques et, notamment, le psoriasis, l'eczéma, le lichen plan, les lésions de la peau associées à un lupus, les dermatites, telles que les dermatites atopique, sébhorréïque ou solaire, les kératoses, telles que la kératose sébhorréïque, sénile, actinique, photo-induite ou folliculaire, l'acné vulgaire, les kéloïdes, les nevi, les verrues, les ichtyoses et les cancers cutanés;
2) les affections inflammatoires ne présentant pas de trouble de la kératinisation telles que l'arthrite;

La présente invention a également pour objet des compositions pharmaceutiques contenant au moins un composé de formule (I) telle que définie ci-dessus, l'un de ses isomères optiques ou géométriques ou un de ses sels.

La présente invention a donc aussi pour objet une composition pharmaceutique destinée notamment au traitement des affections susmentionnées, caractérisée par le fait qu'elle comporte, dans un support pharmaceutiquement acceptable au moins un composé de formule (I), l'un de ses isomères optiques ou géométriques, ou un de ses sels.
D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

L'administration de la composition selon l'invention peut être effectuée par voie entérale, parentérale ou topique. De préférence, la composition pharmaceutique est conditionnée sous une forme convenant à une application par voie topique.

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les composés sont utilisés selon l'invention sont généralement administrés à une dose journalière d'environ 0,001 mg/kg à 100 mg/kg en poids corporel en 1 à 3 prises.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter soit sous forme anhydre, soit sous forme aqueuse.

Les composés sont utilisés par voie topique à une concentration généralement comprise entre 0,001 % et 10 % en poids, de préférence entre 0,01 et 1 % en poids, par rapport au poids total de la composition.

Les compositions telles que décrites précédemment peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; les acides eicosa-5,8,11,14-tétraynoïque et eicosa-5,8,11-triynoïque, leurs esters et amides et enfin les rétinoïdes.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre d'illustration et sans aucun caractère limitatif, plusieurs exemples d'obtention de composés actifs de formule (I) selon l'invention, ainsi que diverses formulations concrètes à base de tels composés. Dans ce qui suit ou ce qui précède, les pourcentages sont donnés en poids sauf mention contraire.

Les différents produits de cette invention sont préparés à partir des intermédiaires halogénés dont la préparation est décrite dans les exemples 1, 6, 9, 13, 16, 18, et 20.

### Exemple 1: Information technique

### Préparation du Chloro-7 thia-3 heptyne-5oate de méthyle

A un solution de 2 ml de thioglycolate de méthyle dans 20ml de méthanol à 10°C sous atmosphère inerte, on ajoute goutte à goutte (pour que la température n'excède pas 15°C) 4.22 ml d'une solution à 30% de méthylate de sodium dans le méthanol. Le mélange est maintenu 30 mn sous agitation puis additionné à une solution de 6.1 ml de dichloro-1,4-butyne-2 dans 25 ml de méthanol sous atmosphère inerte. On maintient sous agitation 6 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le dichloro-1,4-butyne-2 en excès dans l'huile ainsi obtenue est éliminé par distillation sous pression réduite. Le culot de distillation huileux obtenu est chromatographié sur colonne de gel de silice (CH₂Cl₂) conduisant à deux grammes de chloro-7 thia-3 heptyn-5 oate de méthyle sous forme d'huile jaune pâle (Rendement 65%).
**RMN** ^{**1**}**H 200MHz CDCl**_{**3**}: 3.39 (s, 2H), 3.44 (t, 2H), 3.73 (s, 3H), 4.15 (t, 2H).
**RMN** ^{**13**}**C 50 MHz CDCl**_{**3**}: 20.16, 30.45, 32.41, 52.42, 78.25, 81.67, 170.28.

Cl-CH₂-C≡C-CH₂-S-CH₂-CO₂Me

### Exemple 2: Information technique

### Préparation du dithia-3,8 tridécafluoro-11,11,12,12,13,13,14,14,15,15,16,16,16 hexadécyne-5oate de méthyle

A un solution de 3.02g de Foralkyl EM6 dans 30ml de méthanol sous atmosphère inerte, on ajoute goutte à goutte 1.5 ml d'une solution à 30% de méthylate de sodium dans le méthanol. Le mélange est maintenu 30 mn sous agitation puis additionné à une solution de 1.53g de chloro-7 thia-3 heptyn-5 oate de méthyle dans 10 ml de méthanol sous atmosphère inerte. On maintient sous agitation 12 heures à température ambiante puis 2 heures à 50°C puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. L'huile ainsi obtenue est chromatographiée sur colonne de gel de silice (CH₂Cl₂/Heptane 60/40) conduisant à 2.3 grammes de dithia-3,8 tridécafluoro-11,11,12,12,13,13,14,14,15,15,16,16,16 hexadécyn-5 oate de méthyle sous forme d'huile jaune pâle (Rendement 73.5%).
**RMN** ^{**1**}**H 200 MHz CDCl**_{**3**}: 2.30-2.56 (m, 2H), 2.95-2.86 (m, 2H), 3.34 (t, 2H),3.39 (s, 2H), 3.43 (t, 2H), 3.74 (s, 3H).
**RMN** ^{**13**}**C 50 MHz CDCl**_{**3**}: 19.87, 20.29, 22.24, 31.17, 31.61, 32.05, 32.37, 52.34, 78.61, 170.31 (**1 seul C acétylénique, C porteurs de fluor non sortis**).

C₆F₁₃-CH₂-CH₂-S-CH₂-C ≡ C-CH₂-S-CH₂-CO₂Me

### Exemple 3:

### Préparation de l'acide tridécafluoro-11,11,12,12,13,13,14,14,15,15,16,16,16 dithia-3,8 hexadécyne-5oïque

L'acide est préparé par saponification de l'ester dithia-3,8 tridécafluoro-11,11,12,12,13,13,14,14,15,15,16,16,16 hexadécyn-5 oate de méthyle et purifié par chromatographie rapide sur une courte colonne de gel de silice (CH₂-Cl₂/MeOH). On isole ainsi l'acide sous forme de cire beige avec un rendement de 87%
**RMN** ^{**1**}**H 200 MHz CDCl**_{**3**}: 2.20-2.60 (m, 2H), 2.85-2.93 (m, 2H), 3.34 (t, 2H),3.42 (s, 2H), 3.46 (t, 2H).

| **Analyse élémentaire:** | | C | H | S | F |
|---|---|---|---|---|---|
| | Calculé | 32.19 | 2.12 | 12.28 | 47.28 |
| | Trouvé | 32.32 | 2.11 | 12.36 | 47.27 |

C₆F₁₃-CH₂-CH₂-S-CH₂-C ≡ C-CH₂-S-CH₂-CO₂H

### Exemple 4: Information technique

### Préparation du dithia-3,8 docosyne-5oate de méthyle

A un solution de 665µl de tétradécanethiol dans un mélange 5ml méthanol/2 ml THF sous atmosphère inerte, on ajoute 460µl d'une solution à 30% de méthylate de sodium dans le méthanol. Le mélange est maintenu 30 mn sous agitation puis on additionne 0.47g de chloro-7 thia-3 heptyn-5 oate de méthyle dans 5 ml de méthanol sous atmosphère inerte. On maintient sous agitation 8 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. La cire ainsi obtenue est chromatographiée sur colonne de gel de silice (CH₂Cl₂) conduisant à 1.05 g de dithia-3,8 docosyn-5 oate de méthyle sous forme d'huile. (Rendement quantitatif).
**RMN** ^{**1**}**H 200 MHz CDCl**_{**3**}: 0.88 (s, 3H), 1.15-1.50 (m, 22H), 1.50-1.75 (m, 2H), 2.66 (t, 2H), 2.28 (m, 2H), 3.42 (s, 2H), 3.43-3.47 (m, 4H), 3.75 (s, 3H).
**RMN** ^{**13**}**C 100 MHz CDCl**_{**3**}: 14.08, 19.71, 20.63, 22.68, 28.87, 29.09, 29.24, 29.34, 29.53, 29.61, 29.67, 31.81, 31.92, 32.55, 52.40, 77.53, 80.08, 170.47.

C₁₄H₂₉-S-CH₂-C ≡ C-CH₂-S-CH₂-CO₂Me

### Exemple 5:

### Préparation de l'acide dithia-3,8 docosyne-5oïque

L'acide est préparé par saponification de l'ester dithia-3,8 docosyn-5 oate de méthyle et purifié par recristallisation dans l'heptane bouillant. On isole ainsi l'acide dithia-3,8 docosyn-5 oïque sous forme de solide blanc avec un rendement de 81.5%.
**RMN** ^{**1**}**H 200 MHz CDCl**_{**3**}**:** 0.87 (t, 3H), 1.15-1.46 (m, 22H), 1.46-1.76 (m, 2H), 2.65 (t, 2H), 3.28 (s, 2H), 3.46 (m, 4H).
**RMN** ^{**13**}**C 50 MHz CDCl**_{**3**}: 14.01, 19.53, 20.55, 22.58, 28.76, 28.92, 29.14, 29.25, 29.44, 29.56, 31.68, 31.81, 32.27, 80.34, 175.73.

| **Analyse élémentaire:** | | C | H | O | S |
|---|---|---|---|---|---|
| | Calculé | 64.47 | 9.74 | 8.59 | 17.21 |
| | Trouvé | 64.09 | 9.64 | 9.24 | 17.06 |

C₁₄H₂₉-S-CH₂-C ≡ C-CH₂-S-CH₂-CO₂H

### Exemple 6: Information technique

### Préparation du Chloro-1 thia-5 tridécyne-2

A un solution de 5g d'octanethiol dans 60 ml de méthanol sous atmosphère inerte, on ajoute goutte à goutte 6.46ml d'une solution à 30% de méthylate de sodium dans le méthanol. Le mélange est maintenu 30 mn sous agitation puis additionné à 9.35ml de dichloro-1,4-butyne-2 dans 70 ml de méthanol sous atmosphère inerte. On maintient sous agitation 12 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le dichloro-1,4-butyne-2 en excès dans l'huile ainsi obtenue est éliminée par distillation sous pression réduite. Le culot de distillation huileux obtenu est chromatographié sur colonne de gel de silice (CH₂Cl₂) conduisant à 7.5g de chloro-1 thia-5 tridécyne-2 sous forme d'huile jaune pâle (Rendement 94%).
**RMN** ^{**1**}**H 200 MHz CDCl**_{**3**}: 0.82 (s, 3H), 1.1-1.3 (m, 10H), 1.30-1.65 (m, 2H), 2.65 (t, 2H), 3.22 (t, 2H), 4.15 (t, 2H).
**RMN** ^{**13**}**C 50 MHz CDCl**_{**3**}:14.21, 19.62, 19.83, 22.77, 28.93, 29.11, 29.30, 30.87, 31.92, 77.45, 83.25.

C₈H₁₇-S-CH₂-C ≡ C-CH₂-Cl

### Exemple 7: Information technique

### Préparation du dithia-3,8 hexadécyne-5oate de méthyle

A un solution de 980 µl de thioglycolate de méthyle dans 10ml de méthanol à 10°C sous atmosphère inerte, on ajoute goutte à goutte (pour que la température n'excède pas 15°C) 2.03 ml d'une solution à 30% de méthylate de sodium dans le méthanol. Le mélange est maintenu 30 mn sous agitation puis additionné à une solution de 2.5g de chloro-1 thia-5 tridécyne-2 dans un mélange de 7 ml de méthanol avec 3 ml de THF sous atmosphère inerte. On maintient sous agitation 15 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le résidu huileux obtenu est chromatographié sur colonne de gel de silice (CH₂Cl₂) conduisant à 2.3 grammes de dithia-3,8 hexadécyn-5 oate de méthyle sous forme d'huile orange pâle (Rendement 71%).
**RMN** ^{**1**}**H 200 MHz CDCl**_{**3**}: 0.81 (t, 3H), 0.90-1.50 (m, 10H), 1.50-1.61 (m, 2H), 2.62 (t, 2H), 3.25 (t, 2H), 3.39 (s, 2H), 3.41 (t, 2H), 3.72 (s, 3H).
**RMN** ^{**13**}**C 50 MHz CDCl**_{**3**}: 14.0, 19.53, 20.47, 22.54, 28.75, 28.92, 29.08, 31.61, 31.70, 32.35, 52.35, 77.38, 79.91, 170.38.

C₈H₁₇-S-CH₂-C ≡ C-CH₂-S-CH₂-CO₂Me

### Exemple 8:

### Préparation de l'acide dithia-3,8 hexadécyne-5oïque

L'acide est préparé par saponification de l'ester dithia-3,8 hexadécyn-5 oate de méthyle et purifié par recristallisation dans l'heptane bouillant puis dans l'éther diisopropylique. On isole ainsi l'acide dithia-3,8 hexadécyn-5 oïque sous forme de solide beige avec un rendement de 67%.
**RMN** ^{**1**}**H 200 MHz CDCl**_{**3**}:0.84 (t, 3H), 1.1-1.45 (m, 10H), 1.45-1.7 (m, 2H), 2.62 (t, 2H), 3.25 (t, 2H), 3.41 (s, 2H), 3.43 (t, 2H).
**RMN** ^{**13**}**C 50 MHz CDCl**_{**3**}: 14.09, 19.61, 20.63, 22.64, 28.84, 29.00, 29.17, 31.76, 32.35, 80.55, 176.01.

| **Analyse élémentaire:** | | C | H | O | S |
|---|---|---|---|---|---|
| | Calculé | 58.29 | 8.39 | 11.09 | 22.23 |
| | Trouvé | 58.57 | 8.44 | 11.26 | 21.94 |

C₈H₁₇-S-CH₂-C ≡ C-CH₂-S-CH₂-CO₂H

### Exemple 9: Information technique

### Préparation du Bromo-1 tridécyne-2

Le bromo-1 tridécyne-2 (huile incolore) est préparé à partir du tridécyn-2-ol-1 en utilisant le mélange CBr₄/triphénylphosphine dans le dichlorométhane pour réaliser l'halogénation. On forme ainsi le bromo-1 tridécyne-2 (huile incolore) avec un rendement de 91%.
**RMN** ^{**1**}**H 200 MHz CDCl**_{**3**}: 0.84 (t, 3H), 1.15-1.57 (m, 16H), 2.20 (t.t, 2H), 3.89 (t, 2H).

C₁₀H₂₁-C ≡ C-CH₂-Br

### Exemple 10:

### Préparation de l'acide thia-3 hexadécyne-5oïque

A un solution de 422 µl d'acide thioglycolique dans 5ml de méthanol sous atmosphère inerte, on ajoute goutte à goutte 2.20 ml d'une solution à 30% de méthylate de sodium dans le méthanol. Le mélange est maintenu 30 mn sous agitation puis additionné à une solution de 1.5g de bromo-1 tridécyne-2 dans 10 ml de méthanol sous atmosphère inerte. On maintient sous agitation 15 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le résidu huileux obtenu cristallise au refroidissement.

L'acide thia-3 hexadécyn-5 oïque est recristallisé dans l'heptane puis dans l'éther diisopropylique et isolé sous forme de paillettes blanches avec un rendement de 25%.
**RMN** ^{**1**}**H 200 MHz CDCl**_{**3**}: 0.87 (t, 3H), 1.1-1.65 (m, 16H), 2.19 (m, 2H), 3.42 (t, 2H), 3.46 (s, 2H).
**RMN** ^{**13**}**C 50 MHz CDCl**_{**3**}: 14.11, 18.78, 20.81, 22.68, 28.72, 28.89, 29.12, 29.31, 29.54, 31.90, 32.31, 74.16, 85.04, 175.44.

| **Analyse élémentaire:** | | C | H | O | S |
|---|---|---|---|---|---|
| | Calculé | 66.62 | 9.69 | 11.83 | 11.86 |
| | Trouvé | 66.77 | 9.64 | 12.05 | |

C₁₀H₂₁-C ≡ C-CH₂-S-CH₂-CO₂H

### Exemple 11: Information technique

### Préparation du dithia-3,8 heptadécyne-5oate de méthyle

A un solution de 2ml de nonanethiol dans un mélange 20ml méthanol/5 ml THF sous atmosphère inerte, on ajoute 2ml d'une solution à 30% de méthylate de sodium dans le méthanol. Le mélange est maintenu 30 mn sous agitation puis additionné à une solution de 2g de chloro-7 thia-3 heptyn-5 oate de méthyle dans 20 ml de méthanol sous atmosphère inerte. On maintient sous agitation 15 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. L'huile ainsi obtenue est chromatographiée sur colonne de gel de silice (CH₂Cl₂/Heptane 85/15) conduisant à 2.1 g de dithia-3,8 heptadécyn-5 oate de méthyle sous forme d'huile orangée. (Rendement 64%).
**RMN** ^{**1**}**H 200 MHz CDCl**_{**3**}: 0.83 (t, 3H), 1.0-1.65 (m, 14H), 2.64 (t, 2H), 3.26 (t, 2H), 3.39 (s, 2H), 3.42 (t, 2H), 3.73 (s, 3H).
**RMN** ^{**13**}**C 50 MHz CDCl**_{**3**}: 14.11, 19.64, 20.58, 22.66, 28.85, 29.02, 29.25, 29.48, 31.71, 31.86, 32.46, 52.46, 77.47, 80.02, 170.50.

C₉H₁₉-S-CH₂-C ≡ C-CH₂-S-CH₂-CO₂Me

### Exemple 12:

### Préparation de l'acide dithia-3,8 heptadécyne-5oïque

L'acide est préparé par saponification de l'ester dithia-3,8 heptadécyn-5 oate de méthyle et purifié par recristallisation dans l'éther diisopropylique. On isole ainsi l'acide dithia-3,8 heptadécyn-5 oïque sous forme de solide blanc avec un rendement de 44%.
**RMN** ^{**1**}**H 200 MHz CDCl**_{**3**}: 0.84 (t, 3H), 1.1-1.45 (m, 12H), 1.45-1.7 (m, 2H), 2.65 (t, 2H), 3.27 (t, 2H), 3.44 (s, 2H), 3.46 (t, 2H).
**RMN** ^{**13**}**C 50 MHz CDCl**_{**3**}: 14.10, 19.60, 20.62, 22.65, 28.83, 28.99, 29.22, 29.46, 31.74, 31.90, 32.35, 80.41, 176.10.

| **Analyse élémentaire:** | | C | H | O | S |
|---|---|---|---|---|---|
| | Calculé | 59.56 | 8.66 | 10.58 | 21.20 |
| | Trouvé | 59.75 | 8.70 | 10.42 | 20.96 |

C₉H₁₉-S-CH₂-C ≡ C-CH₂-S-CH₂-CO₂H

### Exemple 13: Information technique

### Préparation du Chloro-1 tétradécadiyne-2,5

A une solution de 5 grammes de décyne-1 dans 15 ml de THF anhydre sous atmosphère inerte, on ajoute goutte à goutte à température ambiante 38 ml de bromure d'éthylmagnésium en solution 1M dans le THF. L'addition terminée, on laisse sous agitation 30mn à température ambiante puis on porte à reflux pendant 1h30. On refroidit à température ambiante puis on ajoute 286 mg de chlorure cuivreux et on porte à nouveau à reflux pendant 1 heure. On refroidit alors entre 40 et 50°C et on ajoute 12.5g de dichloro-1,4 butyne-2 en solution dans 25 ml de THF anhydre. On porte à reflux 1 heure puis on laisse sous agitation 15 h à température ambiante avant de porter à reflux encore 2 h. Le milieu réactionnel est alors refroidi à 4°C et hydrolysé avec précaution par une solution aqueuse saturée de NH₄Cl. Le milieu est alors extrait 3 fois par de l'éther diéthylique et les phases organiques réunies sont lavées 3 fois par de l'eau puis par une solution aqueuse saturée de NaCI avant d'être séchées sur Na₂SO₄, filtrées et concentrées sous vide au rotavapor. Le résidu huileux renfermant du dichloro-1,4 butyne-2 en excès est purifié par distillation sous pression réduitepour conduire au chloro-1 tétradécadiyne-2,5 .(Teb = 111-114°C, 0.36mbar) sous forme d'huile orangée (Rendement 52.4%).
**RMN** ^{**1**}**H 200 MHz CDCl**_{**3**}**:** 0.87 (t, 3H), 1.1-1.5 (m, 12H), 2.13 (t.t, 2H), 3.19 (m, 2H), 4.11 (t, 2H).
**RMN** ^{**13**}**C 50 MHz CDCl**_{**3**}: 9.92, 14.05, 18.62, 22.62, 28.62, 28.84, 29.06, 19.14, 30.68, 31.79, 72.68, 74.87, 81.39, 81.71.

C₈H₁₇-C ≡ C-CH₂- C ≡ C -CH₂-Cl

### Exemple 14:

### Préparation de l'acide thia-3 heptadécadiyne-5,8oïque

A un solution de 372 µl d'acide thioglycolique dans 5ml de méthanol sous atmosphère inerte, on ajoute goutte à goutte 2.02 ml d'une solution à 30% de méthylate de sodium dans le méthanol. Le mélange est maintenu 30 mn sous agitation puis on additionne une solution de 1.2g de chloro-1 tétradécadiyne-2,5 dans 6 ml de méthanol sous atmosphère inerte. On maintient sous agitation 20 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le résidu huileux obtenu cristallise au refroidissement. L'acide thia-3 heptadécadiyn-5,8 oïque est recristallisé dans l'heptane puis dans l'hexane et enfin dans l'éther diisopropylique. On isole ainsi l'acide sous forme de cristaux beige avec un rendement de 49.4%.
**RMN** ^{**1**}**H 200 MHz CDCl**_{**3**}: 0.84 (t, 3H), 1.05-1.50 (m, 12H), 2.13 (m, 2H), 3.17 (m, 2H), 3.42 (t, 2H), 3.45 (t, 2H), 9.75 (s large, 1 H)..
**RMN** ^{**13**}**C 50 MHz CDCl**_{**3**}: 9.88, 14.08, 18.66, 20.53, 22.64, 28.68, 28.88, 29.09, 29.17, 31.82, 32.36, 73.32, 74.62, 79.13, 81.17, 176.21.

| **Analyse élémentaire:** | | C | H | O | S |
|---|---|---|---|---|---|
| | Calculé | 68.53 | 8.63 | 11.41 | 11.43 |
| | Trouvé | 67.88 | 8.59 | 12.02 | 12.21 |

C₈H₁₇-C ≡ C-CH₂- C ≡ C -CH₂-S-CH₂-CO₂H

### Exemple 15: Information technique

### Préparation du Thia-3 heptadécadiyne-5.8 ol-1

A une solution de 374µl de mercapto-2 éthanol dans 5 ml de méthanol anhydre sous atmosphère inerte, on ajoute goutte à goutte à température ambiante 1.06 ml de solution à 30% de méthylate de sodium dans le méthanol. Le mélange est maintenu 30 mn sous agitation puis additionné à une solution de 1.2g de chloro-1 tétradécadiyne-2,5 dans 6 ml de méthanol sous atmosphère inerte. On maintient sous agitation 15 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. L'huile ainsi obtenue cristallise au refroidissement. Elle est purifiée par recristallisation dans un mélange heptane/pentane puis dans un mélange heptane/pentane/diisopropyléther. On isole ainsi le thia-3 heptadécadiyne-5,8 ol-1 sous forme de paillettes jaune pâle avec un rendement de 61%.
**RMN** ^{**1**}**H 200 MHz CDCl**_{**3**}: 0.86 (t, 3H), 1.1-1.6 (m, 12H), 2.12 (m, 2H), 2.89 (t, 2H), 3.15 (m, 2H), 3.26 (t, 2H), 3.78 (t, 2H).
**RMN** ^{**13**}**C 50 MHz CDCl**_{**3**}:9.84, 14.06, 18.63, 19.39, 22.61, 28.65, 28.85, 29.06, 29.14, 31.79, 34.84, 60.24, 73.36, 75.86, 78.24, 81.10.

C₈H₁₇-C ≡ C-CH₂- C ≡ C -CH₂-S-CH₂-CH₂-OH

### Exemple 16: Information technique

### Préparation du Chloro-1 pentadécadiyne-2,5

A une solution de 5 grammes de undécyne-1 dans 15 ml de THF anhydre sous atmosphère inerte, on ajoute goutte à goutte à température ambiante 34.5 ml de bromure d'éthylmagnésium en solution 1M dans le THF. L'addition terminée, on laisse sous agitation 30mn à température ambiante puis on porte à reflux pendant 1h30. On refroidit à température ambiante puis on ajoute 260 mg de chlorure cuivreux et on porte à nouveau à reflux pendant 1 heure. On refroidit alors à température ambiante et on ajoute assez rapidement 11.3g de dichloro-1,4 butyne-2. On porte à reflux 1 h30 puis on laisse sous agitation 15 h à température ambiante avant de porter à reflux encore 3h. Le milieu réactionnel est alors refroidi à 4°C et hydrolysé avec précaution par une solution aqueuse saturée de NH₄Cl. Le milieu est alors extrait 3 fois par de l'éther diéthylique et les phases organiques réunies sont lavées 3 fois par de l'eau puis par une solution aqueuse saturée de NaCI avant d'être séchées sur Na₂SO₄, filtrées et concentrées sous vide au rotavapor. Le résidu huileux renfermant du dichloro-1,4 butyne-2 en excès est purifié par distillation sous pression réduite pour conduire au chloro-1 pentadécadiyne-2,5 sous forme d'huile incolore avec un rendement de 37.8%.
**RMN** ^{**1**}**H 200 MHz CDCl**_{**3**}: 0.87 (t, 3H), 1.1-1.55 (m, 14H), 2.14 (m, 2H), 3.19 (m, 2H), 4.13 (t, 2H).
**RMN** ^{**13**}**C 50 MHz CDCl**_{**3**}: 9.96, 14.10, 18.66, 22.66, 28.64, 28.86, 29.13, 29.27, 29.47, 30.73, 31.86, 72.67, 74.91, 80.86, 81.76.

C₉H₁₉-C ≡ C-CH₂- C ≡ C -CH₂-Cl

### Exemple 17:

### Préparation de l'acide thia-3 octadécadiyne-5,8oïque

A un solution de 611 µl d'acide thioglycolique dans 7ml de méthanol sous atmosphère inerte, on ajoute goutte à goutte 3.2 ml d'une solution à 30% de méthylate de sodium dans le méthanol. Le mélange est maintenu 30 mn sous agitation puis additionné à une solution de 2g de chloro-1 pentadécadiyne-2,5 dans 20 ml de méthanol sous atmosphère inerte. On maintient sous agitation 20 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le résidu huileux obtenu cristallise au refroidissement. L'acide thia-3 octadécadiyn-5,8 oïque est recristallisé dans l'éther diisopropylique et ainsi isolé sous forme de solide blanc avec un rendement de 26%.
**RMN** ^{**1**}**H 200 MHz CDCl**_{**3**}: 0.87 (t, 3H), 1.1-1.6 (m, 14H), 2.13 (t.t, 2H), 3.17 (m, 2H), 3.42 (t, 2H), 3.45 (s, 2H), 10.78 (s large, 1H).
**RMN** ^{**13**}**C 50 MHz CDCl**_{**3**}: 9.87, 14.10, 18.65, 20.52, 22.66, 28.67, 29.13, 29.26, 29.46, 31.85, 32.35, 73.30, 74.62, 79.11, 81.16, 176.25.

| **Analyse élémentaire:** | | C | H | O | S |
|---|---|---|---|---|---|
| | Calculé | 69.34 | 8.90 | 10.87 | 10.89 |
| | Trouvé | 69.17 | 8.91 | 10.70 | 10.66 |

C₉H₁₉-C ≡ C-CH₂-C ≡ C-CH₂- S-CH₂-CO₂H

### Exemple 18: Information technique

### Préparation du Chloro-1 dodécadiyne-2,5

A une solution de 4 grammes de octyne-1 dans 15 ml de THF anhydre sous atmosphère inerte, on ajoute goutte à goutte à température ambiante 38.1 ml de bromure d'éthylmagnésium en solution 1M dans le THF. L'addition terminée, on laisse sous agitation 30mn à température ambiante puis on porte à reflux pendant 1h30. On refroidit à température ambiante puis on ajoute 287 mg de chlorure cuivreux et on porte à nouveau à reflux pendant 1 heure. On refroidit alors à température ambiante et on ajoute au goutte à goutte rapide 9.95ml de dichloro-1,4 butyne-2 en solution dans 20 ml de THF anhydre. On laisse sous agitation 30 mn à température ambiante puis on porte à reflux 2 heures puis on laisse sous agitation 15 h à température ambiante avant de porter à reflux encore 2 h. Le milieu réactionnel est alors refroidi à 4°C et hydrolysé avec précaution par une solution aqueuse saturée de NH₄Cl. Le milieu est alors extrait 3 fois par de l'éther diéthylique et les phases organiques réunies sont lavées 3 fois par de l'eau puis par une solution aqueuse saturée de NaCI avant d'être séchées sur Na₂SO₄, filtrées et concentrées sous vide au rotavapor. Le résidu huileux renfermant du dichloro-1,4 butyne-2 en excès est purifié par distillation sous pression réduite et le chloro-1 dodécadiyne-2,5 est isolé sous forme d'huile jaune pâle (Rendement 36%).
**RMN** ^{**1**}**H 200 MHz CDCl**_{**3**}: 0.88 (t, 3H), 1.1 - 1.5 (m, 8H), 2.14 (t.t, 2H), 3.20 (m, 2H), 4.13 (t, 2H).
**RMN** ^{**13**}**C 50 MHz CDCl**_{**3**}: 9.97, 14.04, 18.66, 22.53, 28.53, 28.60, 30.74, 31.31, 72.71, 74.89, 81.46, 81.76.

C₆H₁₃-C ≡ C-CH₂- C ≡ C -CH₂-Cl

### Exemple 19:

### Préparation de l'acide thia-3 pentadécadiyne-5,8oïque

A un solution de 742 µl d'acide thioglycolique dans 8ml de méthanol sous atmosphère inerte, on ajoute goutte à goutte 3.85 ml d'une solution à 30% de méthylate de sodium dans le méthanol. Le mélange est maintenu 30 mn sous agitation puis additionné à une solution de 2g de chloro-1 dodécadiyne-2,5 dans 20 ml de méthanol sous atmosphère inerte. On maintient sous agitation 15 heures à température ambiante puis le milieu réactionnel est versé sur 100 ml d'eau acide (98ml d'eau+2ml H₂SO₄ concentré) puis extrait 3 fois par de l'éther éthylique. Les phases organiques réunies sont lavées 3 fois par de l'eau puis par une solution aqueuse saturée de NaCI avant d'être séchées (Na₂SO₄), filtrées et concentrées sous vide au rotavapor. Le résidu huileux obtenu cristallise à froid. L'acide thia-3 pentadécadiyn-5,8 oïque est recristallisé dans l'éther diisopropylique.

C₆H₁₃-C ≡ C-CH₂- C ≡ C -CH₂- S-CH₂-CO₂H

### Exemple 20: Information technique

### Préparation du Chloro-1 undécadiyne-2,5

A une solution de 5 grammes d'heptyne-1 dans 15 ml de THF anhydre sous atmosphère inerte, on ajoute goutte à goutte à température ambiante 54.6 ml de bromure d'éthylmagnésium en solution 1M dans le THF. L'addition terminée, on laisse sous agitation 30mn à température ambiante puis on porte à reflux pendant 1h30. On refroidit à température ambiante puis on ajoute 412 mg de chlorure cuivreux et on porte à nouveau à reflux pendant 1 heure. On refroidit alors à température ambiante et on ajoute assez rapidement 14.2ml de dichloro-1,4 butyne-2. On porte à reflux 1h30 puis on laisse sous agitation 15 h à température ambiante avant de porter à reflux encore 2h30. Le milieu réactionnel est alors refroidi à 4°C et hydrolysé avec précaution par une solution aqueuse saturée de NH₄Cl. Le milieu est alors extrait 3 fois par de l'éther diéthylique et les phases organiques réunies sont lavées 3 fois par de l'eau puis par une solution aqueuse saturée de NaCI avant d'être séchées sur Na₂SO₄, filtrées et concentrées sous vide au rotavapor. Le résidu huileux renfermant du dichloro-1,4 butyne-2 en excès est purifié par distillation sous pression réduite pour conduire à 6.55g de chloro-1 undécadiyne-2,5 sous forme d'huile jaune pâle (Rendement 69%).
**RMN** ^{**1**}**H 200 MHz CDCl**_{**3**}: 0.88 (t, 3H), 1.1 - 1.5 (m, 6H), 2.13 (t.t, 2H), 3.19 (m, 2H), 4.13 (t, 2H).
**RMN** ^{**13**}**C 50 MHz CDCl**_{**3**}**:** 9.92, 13.94, 18.58, 22.17, 28.32, 30.71, 31.02, 72.68, 74.86, 81.38, 81.72.

C₅H₁₁-C ≡ C-CH₂- C ≡ C -CH₂-Cl

### Exemple 21 :

### Préparation de l'acide thia-3 heptadécatriyne-5,8,11oïque

Cette synthèse est réalisée en quatre étapes :
- La première étape consiste à préparer à partir de l'heptyne commercial le chloro-1 undécadiyn-2,5 par condensation du dichloro-1,4 butyne (voir l'exemple 20).
- A la seconde étape, le tétradécatriyn-2,5,8 ol est obtenu par réaction du dianion de l'alcool propargylique sur le choro-1 undécadiyn-2,5.
- A la troisième étape, le tétradécatriyn-2,5,8 ol est transformé en bromure correspondant par action du tribromure de phosphore.
- Enfin, à la quatrième étape, ce bromure est mis en réaction avec le dianion de l'acide thioglycolique.

### a)Préparation du tétradécatriyne-2,5,8ol Information technique

Le dianion de l'alcool propargylique est préparé par échange des protons acides (alcool et acétylènique) avec le chlorure de propyle magnésium. Une solution diluée de 4,8 cm³ d'alcool propargylique (0,082 Mole) diluée par 10 cm³ de T.H.F. anhydre est ajoutée goutte à goutte à une suspension contenant 2,1 équivalents de chlorure de propylmagnésium agitée à 0° sous atmosphère inerte dans 100 cm³ de T.H.F. Cet organomagnésien (0,17 Mole) est préparé en faisant réagir 14 cm³ de chloropropane avec 4,2 g de magnésium dans le T.H.F.

Le dégagement de propane ayant cessé, on laisse la température s'élever jusqu'à 20°C puis on porte alors le mélange à la température d'ébullition du solvant pendant 1 h 30. On ajoute alors 0,7 g de cyanure cuivreux qui se solubilise dans le milieu progressivement. On obtient une solution limpide puis à une température de 50° C on ajoute à ce dianion 15 g de chloro-1 undécadiyne-2,5 (0,082 Mole) dilué par 10 cm³ de T.H.F. et ensuite le mélange est porté sous agitation pendant trois heures à la température d'ébullition du solvant - puis abandonné à température ordinaire pendant la nuit.

Ensuite, le mélange réactionnel est versé lentement dans 200 cm³ d'une solution aqueuse 1 N d'acide sulfurique, puis extrait trois fois par 100 cm³ d'acétate d'éthyle. Les phases organiques sont rassemblées, lavées à l'aide d'une solution de chlorure d'ammonium, séchées sur sulfate de magnésium puis l'acétate d'éthyle est éliminé. Le tétradécatriyn-2,5,8 ol brut est dissous dans 150 cm³ d'heptane bouillant.

La solution est alors filtrée, puis refroidie à - 20°C. Les cristaux formés sont rapidement essorés et séchés. On obtient ainsi 7 g de tétradécatriyn -2,5,8 ol sous forme de cristaux beiges.

C₅H₁₁―C≡C―CH₂―C≡C―CH₂―C≡C―CH₂OH

### b)Préparation du bromo-1 tétradécatriyne-2,5,8 Information technique

On transforme directement en bromure correspondant l'alcool obtenu précédemment en ajoutant 2 cm³ de tribromure de phosphore (0,0216 mole) à cet alcool dilué dans 50 cm³ d'éther éthylique. Ce mélange agité sous atmosphère inerte et à l'abri de la lumière est porté à la température d'ébullition du solvant pendant 2 heures puis lavé à température ordinaire à l'aide d'une solution aqueuse saturée de chlorure d'ammonium.

La phase organique est décantée puis séchée sur sulfate de magnésium. Trois heures plus tard, le sulfate de magnésium est éliminé par filtration. Le filtrat contenant le bromo-1 tetradécatriyn-2,5,8 est utilisé directement à l'étape suivante.

C₅H₁₁―C ≡ C―CH₂―C ≡ C―CH₂―C ≡ C―CH₂ Br

### c) Préparation de l'acide thia-3 heptadecatriyne-5,8,11oïque

Au filtrat ainsi obtenu, on ajoute sous agitation et sous atmosphère inerte, une solution contenant 0,0346 mole du dianion de l'acide thioglycolique. Ce dianion étant préparé au préalable en traitant à température ordinaire et sous atmosphère inerte 2,4 cm³ d'acide thioglycolique (0,0346 Mole) dissous dans 50 cm³ de méthanol par 4,2 g de méthanolate de sodium (0,076 mole).

Une heure après l'ajout de ce dianion, à la solution contenant le bromo-1 tétradécatriyn-2,5,8 ce dernier est totalement transformé.

Le mélange réactionnel est versé dans une solution de 350 cm³ d'acide sulfurique 1 N glacé. Le mélange est extrait trois fois à l'éther éthylique. Les phases éthérées sont lavées à l'eau, séchées sur sulfate de sodium puis concentrées.

L'acide thia-3 heptadécatriyn-5,8,11 oïque brut ainsi obtenu sous forme d'un liquide visqueux est dissous dans 100 cm³ d'éther isopropylique. A la solution obtenue, on ajoute du noir animal, le mélange est agité pendant un quart d'heure à température ordinaire puis filtré. Le filtrat est concentré à environ 40 cm³ et de l'heptane est ajouté jusqu'à l'apparition d'un trouble. Le mélange est alors refroidi à - 5°C. Les cristaux formés sont rapidement filtrés, séchés et conservés à 0°C. On obtient 3 g d'acide thia-3 heptadécatriyn-2,5,8 oïque de couleur beige.

Les spectres de ¹H et ¹³C RMN sont conformes à la structure.
**RMN** ^{**1**}**H 80 MHz CDCl**_{**3**} : 0.80 (t, 3 H), 1.1 - 1.65 (m, 6H), 2.15 (t.t., 2 H), 3.16 (s, 4), 3.44 (s, 4 H), 10.0 - 11.0 (ma, H).
**RMN**^{**13**}**C 100 MHz CDCl**_{**3**} **:** 9.76, 9.95, 14.00, 18.67, 20.52, 22.22, 28.42, 31.08, 32.48, 73.55, 73.75, 75.09, 75.21, 78.33, 81.06, 176.47.

C₅ H₁₁―C≡C―CH₂―C≡C―CH₂―C≡C―CH₂―S―CH₂―CO₂H

### Exemple 22:

Dans cet exemple, on a illustré diverses formulations concrètes à base des composés selon l'invention.

### A- VOIE ORALE

(a) Comprimé de 0,2 g
   - Composé de l'exemple 2 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 3 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé de l'exemple 5 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 15 0,05 g
   - Glycérine 1,000g
   - Sorbitol à 70% 1,000g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 10 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 8 0,300 g
   - Vaseline blanche codex 100 g
(c) Crème Eau-dans-Huile non ionique
   - Composé de l'exemple 7 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 4 0,100 g
   - Polyéthylène glycol (PEG 400) 69,900 g
   - Ethanol à 95% 30,000 g
(e) Onguent hydrophobe
   - Composé de l'exemple 14 0,300 g
   - Mirystate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 cst" vendu par GOLDSCHMIDT) 100g
(f) Crème Huile-dans-Eau non ionique
   - Composé de l'exemple 4 0,500 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile 100 g

### Exemple 23

Dans cet exemple on a illustré plusieurs de résultats de tests biologiques des composés de l'invention, ainsi que d'exemples comparatifs.

Les tests biologiques effectués correspondent à ceux décrits dans la demande. La méthode utilisée pour déterminer les AC50 est celle décrite dans Kliewer et al., Nature 358, 771-774, 1992. Ainsi, le pouvoir activateur via PPAR-α, PPAR-γ ou PPAR-δ, de molécules peut être évalué avec un test de transactivation dans lequel les cellules HeLa ont été cotransfectées par un vecteur d'expression codant pour ces récepteurs et un plasmide rapporteur contenant un élément de réponse PPRE cloné en amont d'une partie d'un promoteur du virus SV40 et du gène luciférase. Les cellules cotransfectées sont traitées pendant 24 heures avec les molécules à tester et l'activité de la luciférase est déterminée par luminescence.
La référence 1, molécule de référence des PPAR-α est l'acide [4-Chloro-6(2,3-dimethyl-phenylamino)-pyrimidin-2-ylsulfanyl]acetique;
La référence 2, molécule de référence des PPAR-δ et PPAR-γ est la 5-{4[2-(methyl-pyridin-2-yl-amino)-éthoxy]-benzyl}-thiazolidine-2,4-dione;
Les exemples comparatifs 1 et 2 sont des acides gras insaturés de type thiaeicosa(poly)ynoïque, issus de la demande de brevet européen EP 342115.
L'exemple comparatif 1 est l'acide thia-3eicosatétrayne-5,8,11,14oïque.
L'exemple comparatif 2 est l'acide thia-3eicosatriyne-5,8,11oïque.

Les résultats obtenus dans les tests de transactivation des récepteurs de type PPARs sont regroupés dans le tableau suivant :

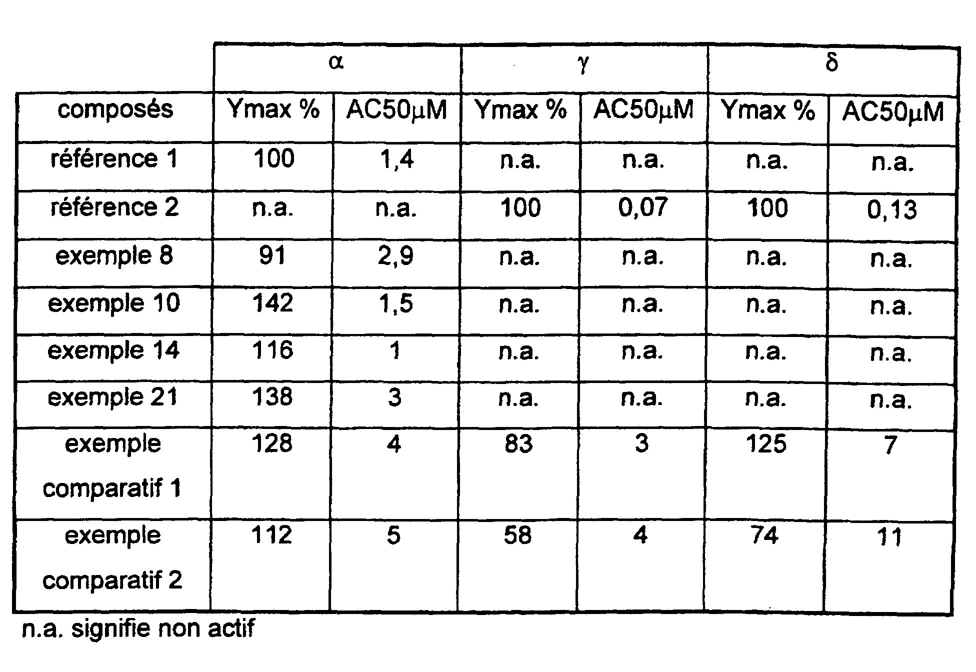

Ces résultats montrent l'activation sélective des composés de l'invention pour les récepteurs de type PPAR-α.
Ces résultats montrent également que des acides gras insaturés de type thiaeicosa(poly)ynoïque, issus de la demande de brevet européen EP 342115 ne présentent pas cette propriété d'activation sélective des récepteurs de type PPAR-α.

## Revendications

1. Composés (poly)thia-alcynoïques, **caractérisés par le fait qu'**ils répondent à la formule (I) suivante :
**R**_{**1**}**-Y-CH**_{**2**}**-C≡C-CH**_{**2**}**-S-CH**_{**2**}**-R**_{**2**} **(I)**
dans laquelle :
- Y représente:
(a) un radical -S(O)t,
t est un nombre entier égal à 0, 1 ou 2,
(b) un radical -CH₂-,
(c) un radical -C ≡ C-,
(d) un radical -C = C-,
- R₁ représente un radical alkyle linéaire ou ramifié ayant de 1 à 18 atomes de carbone éventuellement substitué par un ou plusieurs atomes d'halogène, un radical alkényle linéaire ou ramifié ayant de 1 à 18 atomes de carbone, ou un radical alkynyle linéaire ou ramifié ayant de 1 à 18 atomes de carbone, ce radical pouvant en outre comprendre un ou plusieurs atomes d'oxygène et/ou atomes d'azote et/ou atomes de soufre,
étant entendu que :
- lorsque Y représente (b), alors R₁ comprend un nombre d'atomes compris entre 1 et 12 inclusivement,
- lorsque Y représente (c), alors R₁ comprend un nombre d'atomes compris entre 1 et 10 inclusivement,
- lorsque Y est différent de (b) et que R₁ est un radical insaturé ou comporte un hétéroatome, alors l'insaturation et/ou l'hétéroatome de R₁ ne peuvent pas être en position α par rapport à Y,
- R₂ représente un radical -CO-R₄,
- R₄ représente un radical -OR₅
- R₅ représente un atome d'hydrogène,
et les isomères optiques et géométriques desdits composés de formule (i) ainsi que leurs sels.

2. Composés selon la revendication 1, **caractérisés en ce que**
- lorsque Y représente (b), alors R₁ comprend un nombre d'atomes compris entre 4 et 12 inclusivement,
- lorsque Y représente (c), alors R₁ comprend un nombre d'atomes compris entre 4 et 10 inclusivement.

3. Composés selon l'une des revendications précédentes, **caractérisés en ce que**
- lorsque Y représente (b), alors R₁ comprend un nombre d'atomes compris entre 6 et 12 inclusivement,
- lorsque Y représente (c), alors R₁ comprend un nombre d'atomes compris entre 6 et 10 inclusivement,

4. Composés selon l'une des revendications précédentes, **caractérisés par le fait qu'**ils se présentent sous forme de sels d'un métal alcalin ou alcalino-terreux, de zinc, d'une amine organique ou d'un acide minéral ou organique.

5. Composés selon l'une des revendications précédentes, **caractérisés en ce que** les radicaux alkyles linéaires ou ramifiés ayant de 1 à 18 atomes de carbone éventuellement substitués par un ou plusieurs atomes d'halogène sont choisis parmi les radicaux méthyle, éthyle, propyle, isopropyle, butyle, tertiobutyle, pentyle, hexyle, ou 2-éthyl-hexyle, octyle, nonyle, décyle, dodécyle, dodécanyle, tétradécanyle ou tridécafluoro-3,3,4,4,5,5,6,6,7,7,8,8,8-octyle.

6. Composés selon l'une des revendications précédentes, **caractérisés en ce que** les radicaux alkényles linéaires ou ramifiés ayant de 1 à 18 atomes de carbone sont choisis parmi les radicaux allyle, butènyle, hexènyle, octènyle, décènyle, dodécènyle, ou tétradécènyle.

7. Composés selon l'une des revendications précédentes, **caractérisés en ce que** les radicaux alkynyles linéaires ou ramifiés ayant de 1 à 18 atomes de carbone sont choisis parmi les radicaux propynyle, butyne-2-yle, pentyn-2-yle, hexyn-2-yle, octyn-2-yne, décyn-2-yle, ou dodécyn-2yle-2.

8. Composés selon la revendication 1, **caractérisés par le fait qu'**ils sont pris, seuls ou en mélanges, dans le groupe constitué par :
- l'acide tridécafluoro-11,11,12,12,13,13,14,14,15,15,16,16,16 dithia-3,8 hexadécyne-5oïque,
- l'acide dithia-3,8 docosyne-5oïque,
- l'acide dithia-3,8 hexadécyne-5oïque,
- l'acide thia-3 hexadécyne-5oïque,
- l'acide dithia-3,8 heptadécyne-5oïque,
- l'acide thia-3 heptadécadiyne-5,8oïque,
- l'acide thia-3 octadécadiyne-5,8oïque,
- l'acide thia-3 pentadécadiyne-5,8oïque,
- l'acide thia-3octadecatriyne-5,8,11oïque,
- l'acide thia-3octadecayne-5oïque,
- l'acide thia-3heptadecatriyne-5,8,11oïque,
- l'acide thia-3heptadecayne-5oïque,
- l'acide thia-3hexadecatriyne-5,8,11oïque,
- l'acide thia-3hexadecadiyne-5,8oïque,
- l'acide thia-3pentadecatriyne-5,8,11oïque,
- l'acide thia-3pentadecayne-5oïque,
- l'acide thia-3tetradecayne-5oïque,
- l'acide thia-3 heptadecatriyne-5,8,11oïque

9. Composés selon la revendication 1, **caractérisés par le fait qu'**ils présentent les caractéristiques suivantes :
- R2 est un radical -CO-R4,
- R4 est un radical hydroxyle,
- Y est choisi parmi
- le radical (c) et R1 est un radical alkyle ayant de 4 à 10 atomes de carbone,
ou le radical (a) dans lequel t égal 0 et R1 est un radical alkyle ayant de 4 à 12 atomes de carbone,
ou le radical (b) et R1 est un radical fluoré ayant de 4 à 12 atomes de carbone.

10. Composition cosmétique, **caractérisée par le fait qu'**elle comprend, dans un support cosmétiquement acceptable, au moins l'un des composés tels que définis à l'une quelconque des revendications 1 à 9.

11. Composition selon la revendication 10, **caractérisée en ce que** la concentration en composé(s) selon l'une des revendications 1 à 9 est comprise entre 0,0001 % et 3 % en poids par rapport à l'ensemble de la composition.

12. Utilisation non thérapeutique d'une composition cosmétique telle que définie à l'une des revendications 10 ou 11 pour l'hygiène corporelle ou capillaire.

13. Utilisation non thérapeutique d'une composition cosmétique telle que définie à l'une des revendications 10 ou 11 pour améliorer la fonction barrière cutanée ou promouvoir la différentiation et inhiber la prolifération épidermique.

14. Composés selon l'une quelconque des revendications 1-9 comme médicament.

15. Composés selon la revendication 14 comme médicament destiné au traitement des affections dermatologiques liées à une anomalie de la différenciation des cellules épidermiques et des affections inflammatoires ne présentant pas de trouble de la kératinisation; pour le traitement d'affections inflammatoires.

16. Composés selon la revendication 15, **caractérisé en ce que** les affections dermatologiques liées à une anomalie de la différenciation des cellules épidermiques sont choisies parml le psoriasis, l'eczéma, le lichen plan, les lésions de la peau associées à un lupus, les dermatites, les kératoses, l'acné vulgaire, les kéloïdes, les nevi, les verrues, les ichtyoses et les cancers cutanés; les affections inflammatoires ne présentant pas de trouble de la kératinisation; pour le traitement d'affections inflammatoire sont choisies parmi l'arthrite.

17. Composés selon la revendication 16, **caractérisé en ce que** les dermatites, sont choisies parmi les dermatites atopique, sébhorréïque ou solaire, les kératoses, sont choisies parmi la kératose sébhorréïque, sénile, actinique, photo-induite ou folliculaire.

18. Composition pharmaceutique, **caractérisée par le fait qu'**elle comprend, dans un support pharmaceutiquement acceptable, au moins l'un des composés tels que définis à l'une quelconque des revendications 1 à 9.

19. Composition selon la revendication 18, **caractérisée en ce que** la concentration en composé(s) selon l'une des revendications 1 à 9 est comprise entre 0,001 % et 10 % en poids par rapport à l'ensemble de la composition.

## Patentansprüche

1. (Poly)thiaalkinverbindungen, **dadurch gekennzeichnet, dass** sie der folgenden Formel (I) entsprechen:
R₁-Y-CH₂-C≡C-CH₂-S-CH₂-R₂ (I),
worin bedeuten:
- Y bedeutet:
(a) eine Gruppe -S(O)ₜ, wobei t eine ganze Zahl 0, 1 oder 2 bedeutet,
(b) die Gruppe -CH₂-, ,
(c) die Gruppe -C≡C-,
(d) die Gruppe C=C-,
- die Gruppe R₁ bedeutet eine geradkettige oder verzweigte Alkylgruppe mit 1 bis 18 Kohlenstoffatomen, eine geradkettige oder verzweigte Alkenylgruppe mit 1 bis 18 Kohlenstoffatomen, die gegebenfalls mit einem oder mehreren Halogen atomen substituiert sind, oder eine geradkettige oder verzweigte Alkinylgruppe mit 1 bis 18 Kohlenstoffatomen, wobei die Gruppe ferner ein oder mehrere Sauerstoffatome und/oder Stickstoffatome und/oder Schwefelatome enthalten kann, mit der Maßgabe dass:
- in R₁ die Anzahl der Atome im Bereich von 1 bis 12 liegt, wenn Y (b) bedeutet,
- in R₁ die Anzahl der Atome im Bereich von 1 bis 10 liegt, wenn Y (c) bedeutet,
- sich die ungesättigte Bindung und/oder das Heteroatom in R₁ nicht in α-Stellung zur Gruppe Y befindet, wenn Y von (b) verschieden ist und R₁ eine ungesättigte Gruppe oder ein Heteroatom enthält,
- R₂ eine Gruppe -CO-R₄,
- R₄ eine Gruppe -OR₅,
- R₅ Wasserstoff,
und die optischen und geometrischen Isomere der Verbindungen der Formel (I) sowie deren Salze.

2. Verbindungen nach Anspruch 1, **dadurch gekennzeichnet, dass**
- die Anzahl der Atome von R₁ im Bereich von 4 bis 12 liegt, wenn Y (b) bedeutet,
- in R₁ die Anzahl der Atome im Bereich von 4 bis 10 liegt, wenn Y (c) bedeutet.

3. Verbindungen nach einem der vorhergehenden Ansprüche, **dadurch gekennzeichnet, dass**
- in R₁ die Zahl der Atome im Bereich von 6 bis 12 liegt, wenn Y (b) bedeutet,
- in R₁ die Anzahl der Atome im Bereich von 6 bis 10 liegt, wenn Y (c) bedeutet.

4. Verbindungen nach einem der vorhergehenden Ansprüche, **dadurch gekennzeichnet, dass** sie in Form der Salze mit einem Alkalimetall oder Erdalkalimetall, in Form der Zinksalze, in Form der Salze mit einem organischen Amin oder in Form der Salze mit einer anorganischen oder organischen Säure vorliegen.

5. Verbindungen nach einem der vorhergehenden Ansprüche, **dadurch gekennzeichnet, dass** die geradkettigen oder verzweigten Alkylgruppen mit 1 bis 18 Kohlenstoffatomen, die gegebenenfalls mit einem oder mehreren Halogenatomen substituiert sind, unter Methyl, Ethyl, Propyl, Isopropyl, Butyl, *t*-Butyl, Pentyl, Hexyl, 2-Ethylhexyl, Octyl, Nonyl, Decyl, Dodecyl, Dodecanyl, Tetradecanyl oder 3,3,4,4,5,5,6,6,7,7,8,8,8-Tridecafluoroctyl ausgewählt sind.

6. Verbindungen nach einem der vorhergehenden Ansprüche, **dadurch gekennzeichnet, dass** die geradkettigen oder verzweigten Alkenylgruppen mit 1 bis 18 Kohlenstoffatomen unter Allyl, Butenyl, Hexenyl, Octenyl, Decenyl, Dodecenyl oder Tetradecenyl ausgewählt sind.

7. Verbindungen nach einem der vorhergehenden Ansprüche, **dadurch gekennzeichnet, dass** die geradkettigen oder verzweigten Alkinylgruppen mit 1 bis 18 Kohlenstoffatomen unter Propinyl, 2-Butinyl, 2-Pentinyl, 2-Hexinyl, 2-Octinyl, 2-Decinyl oder 2-Dodecinyl ausgewählt sind.

8. Verbindungen nach Anspruch 1, **dadurch gekennzeichnet, dass** sie unter den folgenden Verbindungen oder deren Gemischen ausgewählt sind:
- 11,11,12,12,13,13,14,14,15,15,16,16,16-Tridecafluor-3,8-dithia-5-hexadecinsäure,
- 3,8-Dithia-5-docosinsäure,
- 3,8-Dithia-5-hexadecinsäure,
- 3-Thia-5-hexadecinsäure,
- 3,8-Dithia-5-heptadecinsäure,
- 3-Thia-5,8-heptadecadiinsäure,
- 3-Thia-5,8-octadecadiinsäure,
- 3-Thia-5,8-pentadecadiinsäure,
- 3-Thia-5,8,11-octadecatriinsäure,
- 3-Thia-5-octadecainsäure,
- 3-Thia-5,8,11-heptadecatriinsäure,
- 3-Thia-5-heptadecainsäure,
- 3-Thia-5,8,11-hexadecatriinsäure,
- 3-Thia-5,8-hexadecadiinsäure,
- 3-Thia-5,8,11-pentadecatriinsäure,
- 3-Thia-5-pentadecainsäure,
- 3-Thia-5-tetradecainsäure, und
- 3-Thia-5,8,11-heptadecatriinsäure.

9. Verbindungen nach Anspruch 1, **dadurch gekennzeichnet, dass** sie die folgenden Eigenschaften aufweisen:
- R₂ ist eine Gruppe -CO-R₄,
- R₄ ist eine Hydroxygruppe,
- Y ist ausgewählt unter:
- der Gruppe (c) und R₁ bedeutet eine Alkylgruppe mit 4 bis 10 Kohlenstoffatomen, oder
der Gruppe (a), worin t 0 bedeutet, und R₁ ist eine Alkylgruppe mit 4 bis 12 Kohlenstoffatomen, oder
der Gruppe (b) und R₁ ist eine fluorierte Gruppe mit 4 bis 12 Kohlenstoffatomen.

10. Kosmetische Zusammensetzung, **dadurch gekennzeichnet, dass** sie in einem kosmetisch akzeptablen Träger mindestens eine der Verbindungen nach einem der Ansprüche 1 bis 9 enthält.

11. Zusammensetzung nach Anspruch 10, **dadurch gekennzeichnet, dass** die Konzentration der Verbindung(en) nach einem der Ansprüche 1 bis 9 im Bereich von 0,0001 bis 3 Gew.-%, bezogen auf die gesamte Zusammensetzung, liegt.

12. Nichttherapeutische Verwendung einer kosmetischen Zusammensetzung nach einem der Ansprüche 10 oder 11 zur Körperpflege oder Haarpflege.

13. Nichttherapeutische Verwendung einer kosmetischen Zusammensetzung nach einem der Ansprüche 10 oder 11 zur Verbesserung der Barrierefunktion der Haut oder zur Förderung der Differenzierung und Inhibierung der Proliferation der Epidermis.

14. Verbindungen nach einem der Ansprüche 1 bis 9 als Arzneimittel.

15. Verbindungen nach Anspruch 14 als Arzneimittel, die zur Behandlung von dermatologischen Erkrankungen, die mit einer anomalen Differenzierung der Epidermiszellen verbunden sind, und entzündlichen Erkrankungen, die keine Keratisierungsstörung zeigen; und zur Behandlung von entzündlichen Erkrankungen vorgesehen sind.

16. Verbindungen nach Anspruch 15, **dadurch gekennzeichnet, dass** die dermatologischen Erkrankungen, die mit einer anomalen Differenzierung der Epidermiszellen verbunden sind, unter Psoriasis, Ekzemen, Lichen planus, Schädigungen der Haut, die mit Lupus einhergehen, Dermatitis, Keratosen, Acne vulgaris, Keloiden, Nevi, Warzen, Ichtyosen und Hautkarzinomen, entzündlichen Erkrankungen, die keine Keratinisierungsstörung zeigen; hinsichtlich der Behandlung von entzündlichen Erkrankungen unter Arthritis ausgewählt sind.

17. Verbindungen nach Anspruch 16, **dadurch gekennzeichnet, dass** die Hautentzündungen unter Neurodermitis, Dermatitis seborrhoica oder Sonnendermatitis und die Keratosen unter Keratosis seborrhoica, Keratosis senilis, Strahlenkeratose, lichtinduzierter Keratose oder Keratosis follicularis ausgewählt sind.

18. Pharmazeutische Zusammensetzung, **dadurch gekennzeichnet, dass** sie in einem pharmazeutisch akzeptablen Träger mindestens eine der Verbindungen nach einem der Ansprüche 1 bis 9 enthält.

19. Zusammensetzung nach Anspruch 18, **dadurch gekennzeichnet, dass** die Konzentration der Verbindung(en) nach einem der Ansprüche 1 bis 9 im Bereich von 0,001 bis 10 Gew.-%, bezogen auf die gesamte Zusammensetzung, liegt.

## Claims

1. (Poly)thiaalkynoic compounds, **characterized in that** they correspond to the following formula (I):
**R**_{**1**}**-Y-CH**_{**2**}**-C≡C-CH**_{**2**}**-S-CH**_{**2**}**-R**_{**2**} **(I)**
in which:
- Y represents:
(a) an -S(O)t radical,
t is an integer equal to 0, 1 or 2,
(b) a -CH₂- radical,
(c) a -C≡C- radical,
(d) a -C=C- radical,
- R₁ represents a linear or branched alkyl radical having from 1 to 18 carbon atoms which are optionally substituted with one or more halogen atoms, a linear or branched alkenyl radical having from 1 to 18 carbon atoms, or a linear or branched alkynyl radical having from 1 to 18 carbon atoms, it being possible for this radical, in addition, to comprise one or more oxygen atoms and/or nitrogen atoms and/or sulphur atoms,
it being understood that:
- when Y represents (b), then R₁ comprises a number of atoms of between 1 and 12 inclusive,
- when Y represents (c), then R₁ comprises a number of atoms of between 1 and 10 inclusive,
- when Y is different from (b) and R₁ is an unsaturated radical or comprises a heteroatom, then the unsaturation and/or the heteroatom of R₁ cannot be at the α position with respect to Y,
- R₂ represents a -CO-R₄ radical,
- R₄ represents an -OR₅ radical,
- R₅ represents a hydrogen atom,
and the optical and geometric isomers of the said compounds of formula (i) as well as their salts.

2. Compounds according to Claim 1, **characterized in that**
- when Y represents (b), then R₁ comprises a number of atoms between 4 and 12 inclusive,
when Y represents (c), then R₁ comprises a number of atoms between 4 and 10 inclusive.

3. Compounds according to one of the preceding claims, **characterized in that**
- when Y represents (b), then R₁ comprises a number of atoms between 6 and 12 inclusive,
- when Y represents (c), then R₁ comprises a number of atoms between 6 and 10 inclusive.

4. Compounds according to one of the preceding claims, **characterized in that** they are provided in the form of salts of an alkali or alkaline-earth metal, of zinc, of an organic amine or of an inorganic or organic acid.

5. Compounds according to one of the preceding claims, **characterized in that** the linear or branched alkyl radicals having from 1 to 18 carbon atoms which are optionally substituted with one or more halogen atoms are chosen from the methyl, ethyl, propyl, isopropyl, butyl, tert-butyl, pentyl, hexyl or 2-ethylhexyl, octyl, nonyl, decyl, dodecyl, dodecanyl, tetradecanyl or 3,3,4,4,5,5,6,6,7,7,8,8,8-tridecafluorooctyl radicals.

6. Compounds according to one of the preceding claims, **characterized in that** the linear or branched alkenyl radicals having from 1 to 18 carbon atoms are chosen from the allyl, butenyl, hexenyl, octenyl, decenyl, dodecenyl or tetradecenyl radicals.

7. Compounds according to one of the preceding claims, **characterized in that** the linear or branched alkynyl radicals having from 1 to 18 carbon atoms are chosen from the propynyl, butyn-2-yl, pentyn-2-yl, hexyn-2-yl, octyn-2-yn, decyn-2-yl or 2-dodecyn-2-yl radicals.

8. Compounds according to Claim 1, **characterized in that** they are taken, alone or in the form of mixtures, from the group consisting of:
- 3,8-dithia-12,11,12,12,13,13,14,14,15,15,16,16,16-tridecafluoro-5-hexadecynoic acid,
- 3,8-dithia-5-docosynoic acid,
- 3,8-dithia-5-hexadecynoic acid,
- 3-thia-5-hexadecynoic acid,
- 3,8-dithia-5-heptadecynoic acid,
- 3-thia-5,8-heptadecadiynoic acid,
- 3-thia-5,8-octadecadiynoic acid,
- 3-thia-5,8-pentadecadiynoic acid,
- 3-thia-5,8,12-octadecatriynoic acid,
- 3-thia-5-octadecaynoic acid,
- 3-thia-5,8,11-heptadecatriynoic acid,
- 3-thia-5-heptadecaynoic acid,
- 3-thia-5,8,11-hexadecatriynoic acid,
- 3-thia-5,8-hexadecadiynoic acid,
- 3-thia-5,8,11-pentadecatriynoic acid,
- 3-thia-5-pentadecaynoic acid,
- 3-thia-5-tetradecaynoic acid,
- 3-thia-5,8,11-heptadecatriynoic acid.

9. Compounds according to Claim 1, **characterized in that** they exhibit the following characteristics:
- R2 is a -CO-R4 radical,
- R4 is a hydroxyl radical,
- Y is chosen from
- the radical (c) and R1 is an alkyl radical having from 4 to 10 carbon atoms,
or the radical (a) in which t equals 0 and R1 is an alkyl radical having from 4 to 12 carbon atoms,
or the radical (b) and R1 is a fluorinated radical having from 4 to 12 carbon atoms.

10. Cosmetic composition, **characterized in that** it comprises, in a cosmetically acceptable carrier, at least one of the compounds as defined in any one of Claims 1 to 9.

11. Composition according to Claim 10, **characterized in that** the concentration of compound(s) according to one of Claims 1 to 9 is between 0.0001% and 3% by weight relative to the whole composition.

12. Non-therapeutic use of a cosmetic composition as defined in either of Claims 10 and 11 for body or hair hygiene.

13. Non-therapeutic use of a cosmetic composition as defined in either of Claims 10 and 11 to improve the skin barrier function or promote differentiation and inhibit epidermal proliferation.

14. Compounds according to any one of Claims 1 to 9 as medicament.

15. Compounds according to Claim 14, as a medicament intended for the treatment of dermatological conditions linked to an abnormality in the differentiation of epidermal cells and of inflammatory conditions exhibiting no keratinization disorder, for the treatment of inflammatory conditions.

16. Compounds according to Claim 15, **characterized in that** the dermatological conditions linked to an abnormality in the differentiation of epidermal cells are chosen from psoriasis, eczema, lichen planus, skin lesions associated with a lupus, dermatites, keratoses, acne vulgaris, keloids, nevi, verrucas, ichthyoses and skin cancers; the inflammatory conditions exhibiting no keratinization disorder; for the treatment of inflammatory conditions are chosen from arthritis.

17. Compounds according to Claim 16, **characterized in that** the dermatites are chosen from atopic, seborrhoeic or solar dermatites, the keratoses are chosen from seborrhoeic, senile, actinic, photoinduced or follicular keratosis.

18. Pharmaceutical composition, **characterized in that** it comprises, in a pharmaceutically acceptable carrier, at least one of the compounds as defined in any one of Claims 1 to 9.

19. Composition according to Claim 18, **characterized in that** the concentration of compound(s) according to one of Claims 1 to 9 is between 0.001% and 10% by weight relative to the whole composition.
